# EUROPEAN PATENT SPECIFICATION

(11) **EP 3 017 776 B1**
(45) Date of publication and mention of the grant of the patent: **24.03.2021**
(21) Application number: 15275227.5
(22) Date of filing: 03.11.2015
(51) Int. Cl.: A61M 25/10, A61B 17/3207, A61L 29/06, A61B 17/22

(54) **MEDICAL BALLOON**
MEDIZINISCHER BALLON
BALLONNET MEDICAL

(30) Priority: 07.11.2014 GB 201419864; 07.11.2014 GB 201419873; 28.05.2015 GB 201509130
(43) Date of publication of application: 11.05.2016
(73) Proprietor: Cook Medical Technologies LLC, Bloomington, IN 47404 (US)
(72) Inventor: Aggerholm, Steen, 4660 St. Heddinge (DK); Lysgaard, Thomas, 2680 Solroed Strand (DK)
(74) Representative: Williams Powell

(56) References cited:
- US-A1- 2003 144 683
- US-A1- 2005 038 383
- US-A1- 2009 234 283
- US-A1- 2010 286 594
- US-A1- 2011 160 756
- US-A1- 2012 283 624

## Description

### Technical Field

The present invention relates to a medical balloon, which in some embodiments may be a scoring or cutting balloon for use for instance in angioplasty or other vessel dilatation procedures. In other embodiments the balloon may have increased resistance to tearing and able to be used at high pressures.

### Background Art

Traditionally, vessel dilatation was effected by open surgery procedures but advances in medical technology have enabled such procedures to be carried out endoluminally, principally by means of expandable medical devices of which angioplasty balloons have proven most effective. Some angioplasty balloons have scoring or cutting elements attached to the outside of the main body portion of the balloon, the cutting elements for instance being in the form of sharp metal blades. Another example provides scoring or cutting elements which are integrally formed with the balloon. The scoring or cutting elements are provided to break up plaque within the vessel and thus recanalise the latter. The balloon acts to keep the blades or elements pressed against the vessel wall during the scoring or cutting process and also acts to dilate the vessel as the process is carried out.

A problem with balloons formed with separate scoring or cutting blades fixed to the balloon is that the manufacturing process is more complex, time consuming and costly. Moreover, the eventual structure generally requires a fixation element to fix the blade to the balloon, which is typically in the form of a base support and adhesives or other bonding agents. The resultant structure can be relatively bulky, leading to limitations in the ability to fold and wrap the balloon to small diameters for delivery purposes. This can result in the balloon being unsuitable for smaller vessels or for the treatment of highly restricted vessels. Moreover, the balloon structure can be relatively rigid, leading to loss of flexibility of the balloon when deflated and as a result reduced trackability through tortuous vasculature.

A cutting or scoring balloon formed with integral cutting or scoring elements can resolve many of the shortcomings of balloons formed with separate scoring or cutting elements. However, in some instances the cutting or scoring elements and/or the overall structure of the balloon can be less rigid when deployed, leading to a reduction in cutting or scoring efficiency.

There is also a risk during angioplasty procedures that the balloon may tear, primarily as a result of the existence or generation of sharp edges of plaque and/or the pressure to which the balloon is inflated to during the process. While a tear which propagates in a longitudinal direction of the balloon tends not to be critical, as the balloon can be removed easily form the patient in one piece and thus replaced, tears which propagate circumferentially can be problematic, particularly if this leads to portions of the balloon snagging within the vessel or breaking off.

Furthermore, scoring or cutting balloons tend to be difficult to detect during imaging as a result of their inherent imaging transparency.

At least some of the problems identified above can be exhibited in balloons used for other than angioplasty procedures

US 2010/286594 A1 describes a balloon catheter provided with cutting elements delivering a bioactive upon inflation of the balloon.

US 2003/144683 A1 describes a balloon having concentrated force regions.

Some examples of cutting or scoring balloons can be found in US-2003/0163148, US-2012/0130407, US-2009/0234283, US-2011/0160756, US-2004/0230178 and US-2003/0153870.

### Disclosure of the Invention

The present invention seeks to provide an improved medical balloon, particularly an improved cutting or scoring balloon and an improved high pressure balloon The invention is solely defined by the appended claims 1-15. Reference in the description to embodiments not covered in the appended claims are to be considered as mere examples not forming part of the present invention.

According to an aspect of the present invention, there is provided a medical balloon including:
a balloon body member made of a first polymer material;
the body member having a balloon wall with a wall thickness and inner and outer wall surfaces, the body member being disposed between first and second balloon ends, the balloon wall also extending along end cones; and
at least one elongate element on the balloon wall extending along the body member between the first and second balloon ends and along the end cones, the at least one elongate element being made of a second polymer material, the second polymer material including an amorphous polymer material; at least a part of the or each elongate element having a flattened outer surface which is substantially smooth with the outer wall surface or which is substantially non-protruding from the balloon wall.

Preferred embodiments include elongate elements including an amorphous polymer material. It has been found that amorphous polymer materials can provide rigidity to the elongate elements, for example to enable them to serve effectively as scoring or cutting elements, and can make the elements easily shaped, for example if they are to be shaped to form strengthening elements. In particular, a surprising feature of amorphous material in preferred embodiments of the invention is that, despite it being more rigid and harder when the balloon is used, the amorphous material becomes very soft and formable when the balloon is heated for being formed, in some cases more formable than the material of the balloon wall, allowing rigid elements to be easily formed in a desired configuration.

Some embodiments, with cutting or scoring elements made of amorphous polymer material provide cutting or scoring elements which are more effective than many prior art unitary scoring balloon structures, primarily due to the fact that the amorphous material can be more rigid than non-amorphous material.

Furthermore, the balloon can also be formed in a unitary manner rather than in separate stages.

A particular advantage of using amorphous polymer material in scoring or cutting elements is that the rigidity of amorphous polymer material can improve the performance of the scoring or cutting elements by over 50%. It can therefore be possible to halve the height of the scoring or cutting elements as compared with some prior art balloons and still obtain an improved performance. Reducing the height of the scoring or cutting elements in turn reduces the profile of the balloon as a whole, making it easier to insert into small and/or tortuous vessels. Grilamid TR 55 is one example of amorphous polymer material which can obtain this improved performance.

Preferably, each of the at least one elongate element has a base which extends to the outer wall surface but not into the balloon wall. This can enable the balloon wall to be strong, for example by being made of a non-amorphous polymer material, while the elongate elements on top of the outer wall surface can serve as cutting or scoring or strengthening elements.

It is preferred that the second polymer material has a greater rigidity than a rigidity of the first polymer material.

Advantageously, the first and second polymer materials are of the same polymer type.

Advantageously, the first and second polymer materials are co-extruded. The co-extrusion of materials of the same polymer type is able to provide a unitary balloon without any weakened transition point between the two materials.

In one embodiment, the first and second polymer materials may include the same polymer, optionally with one of the first and second polymer materials including one of more of: an additive, a structural modification and a blend, modifying its characteristics relative to the other of the first and second polymer materials.

In one embodiment, the first material is or includes a polyamide such as Nylon 12 and the second material is or includes a different polyamide, but preferably of the same polymer type, such as Nylon 6 or Nylon 66.

In one embodiment, the second material is or includes an amorphous polyamide 12-based copolymer such as Grilamid TR55.

In the preferred embodiment, the second material is or includes an amorphous polymer material, preferably an amorphous polyamide material, preferably an amorphous polyamide 12-based copolymer. The second material may be or include an amorphous Nylon material such as amorphous Nylon 12. Amorphous polymer materials, especially amorphous polyamide materials and Nylon materials, can provide very rigid elements which can easily be shaped. In addition, they can easily be co-extruded with a first material of the same polymer type to provide a unitary balloon.

In an embodiment, the second material is or includes an amorphous polymer material, such as amorphous Nylon 12, and the first material is or includes a non-amorphous polymer material which may include the same polymer as the second material, for example Nylon 12.

In embodiments, references to amorphous materials can refer to fully-amorphous materials and references to non-amorphous materials can refer to crystalline or semi-crystalline materials.

In one embodiment, the second polymer material is or includes radiopaque or echogenic material. For this purpose, the second polymer material may include between 50 and 90% by weight of radiopaque or echogenic material, for instance between 60% and 80% by weight of radiopaque or echogenic material or substantially 65% or 80% by weight of radiopaque or echogenic material.

In other embodiments, the second polymer material is radiolucent and/or non-echogenic.

In embodiments, the outer wall surface is generally rounded in a circumferential direction of the body member.

In some embodiments, the second polymer material includes tungsten in an amount of between 50% and 90% by weight, between 60% and 80% by weight, and preferably 80% by weight. Tungsten in this amount may assist with making the elongate elements easily shaped.

The second polymer material may include a mix or blend of radiopaque or echogenic material and polymeric material. In an example, the second polymer material includes at least one of: tungsten, gold, platinum, palladium, barium or bismuth.

It is preferred that the medical balloon includes a plurality of said elongate elements, which may be spaced from one another circumferentially around the balloon body member.

The at least one elongate element may extend generally linearly between the first and second ends; although it is not excluded that the element or elements could extend at least partially circumferentially around the body portion, for instance helically.

Advantageously, the at least one elongate element extends along the first and second ends of the balloon and is flattened at the first and second ends. Having such elements extend to the very ends of the balloon can ensure that they provide strength to the balloon along its entire length, in particular resistance against circumferential tear propagation. Flattening the elements at the ends of the balloon increases the flexibility of the balloon, particularly when deflated, and thus improves trackability of the balloon through the patient's vasculature during deployment.

In practice, the balloon ends will include conical end portions terminating in necks which attach to a balloon catheter.

In some embodiments, each of the at least one elongate element has an inflated elongate element thickness in an inflated condition of the balloon and the balloon wall has an inflated wall thickness in an inflated condition of the balloon, and the inflated elongate element thickness of each of the at least one elongate element is greater than the inflated wall thickness.

The skilled person will appreciate that the terms 'inflated wall thickness' and 'inflated elongate element thickness' refer to thicknesses in an inflated condition of the balloon and do not necessarily mean that the thicknesses themselves are inflated.

In preferred embodiments, the inflated elongate element thickness of each of the at least one elongate elements is at least twice, at least four times or most preferably at least six times the inflated wall thickness. Having this greater thickness is advantageously able to stop circumferential tears from propagating. These ratios can be with respect to the thinnest point of the inflated wall thickness or the thickest point of the inflated wall thickness or an average inflated wall thickness.

The at least one elongate element can have an outer surface which is substantially smooth with the outer wall surface of the balloon body member. Most preferably, the at least one elongate element has an outer surface which is rounded. In some embodiments, the at least one elongate element can have an outer surface which is substantially flush with the outer wall surface of the balloon body.

Embodiments can provide a balloon which effects no scoring or cutting function but which has increased resistance against tear propagation and which can therefore be operated at higher pressures.

The at least one elongate element may have a base which extends to the outer wall surface but not into the balloon wall.

The first and second polymer materials may be of the same polymer type.

The first and second polymer materials may include the same polymer.

One of the first and second polymer materials may include one of more of: an additive, a structural modification and a blend, modifying its characteristics relative to the other of the first and second polymer materials.

The first and second materials may include Nylon, for example Nylon 12.

The at least one elongate element and the balloon body member may be co-extruded.

Also described herein is a medical balloon including a balloon body member made of a first polymer material and being substantially radiolucent; the body member having a balloon wall with a wall thickness and inner and outer wall surfaces, the body member being disposed between first and second balloon ends; and at least one scoring or cutting element extending along the body member between the first and second balloon ends, the at least one scoring or cutting element being made of a second polymer material being or including a radiopaque or echogenic material.

The provision of radiopaque scoring or cutting elements of this nature assists in the visualisation of the balloon and hence of its state and performance. This can be achieved without compromising the performance of the balloon.

The second polymer material may include at least one of: tungsten, gold, platinum, palladium, barium or bismuth.

The second polymer material may include between 50 and 90% by weight of radiopaque or echogenic material.

According to an aspect of the invention, there is provided a method of forming a medical balloon including the steps of:
forming a raw balloon tubing by:
   a) forming a tubing portion made of a first polymer material;
   b) applying on the tubing portion at least one elongate element made
   of a second polymer material, the second polymer material including an amorphous polymer material;
placing the raw balloon tubing in a mold;
heating and inflating the raw balloon tubing in the mold to flatten at least a part of the or each elongate element and to provide the at least a part of the or each elongate element with an outer surface which is substantially smooth with an outer wall surface of the balloon or substantially non-protruding from a balloon wall.

In examples the method includes removing the formed balloon from the mold.

The tubing portion and the at least one elongate element can be coextruded.

Heating and inflating the raw balloon tubing in the mold can include flattening the at least one elongate element and/or providing the at least one elongate element with a rounded outer surface and/or an outer surface that is substantially smooth with an outer wall surface of a formed balloon body member.

The method is preferably for forming a balloon as above.

Other features of the apparatus disclosed herein will become apparent from the following specific description of preferred embodiments.

### Brief Description of the Drawings

Embodiments of the present invention are described below, by way of example only, with reference to the accompanying drawings, in which:
Figure 1 is a schematic diagram of an embodiment of medical balloon;
Figure 2 is a transverse cross-sectional view of an embodiment of raw tubing for forming the balloon of Figure 1;
Figure 3 is a transverse cross-sectional view of an embodiment of a balloon;
Figures 9A to 9B are sketches of an embodiment of raw tubing and a blown balloon according to the teachings herein;
Figure 10 is a view of an embodiment of a balloon;
Figure 11 is a transverse cross-sectional view of an embodiment of a balloon in a delivery device;
Figure 12 is a schematic diagram of a mold for use in an embodiment of the invention;
Figure 13 show cross-sections of a part of a balloon with non-amorphous ribs and a part of a balloon with amorphous ribs;

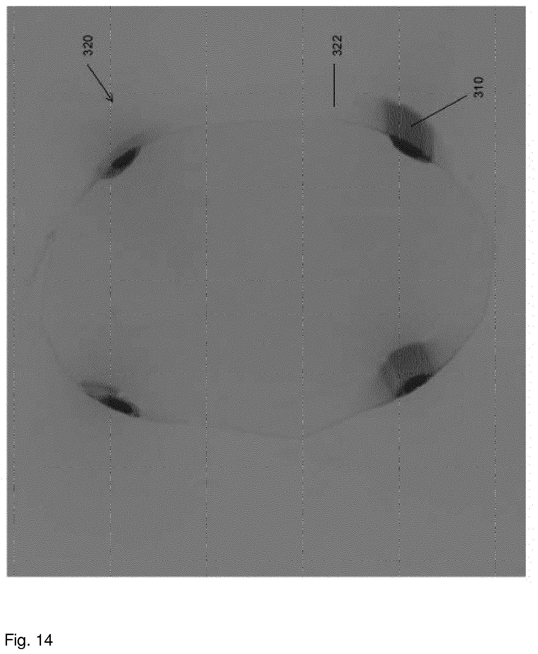

Figure 14 is a photograph corresponding to the view of Figure 10;

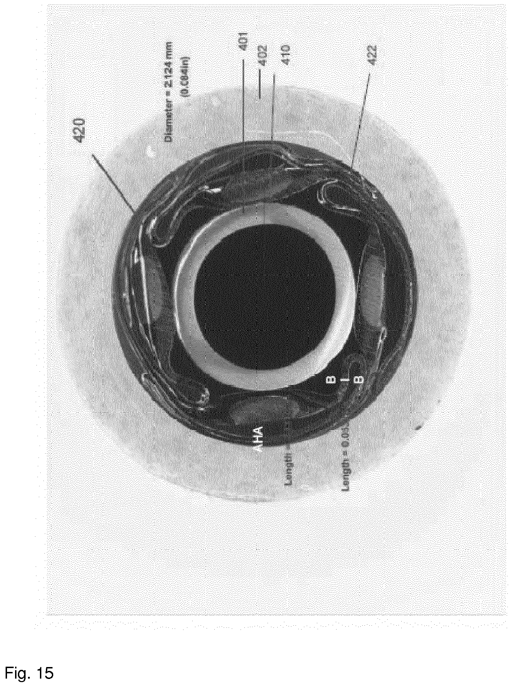

Figure 15 is a photograph corresponding to the view of Figure 11; and

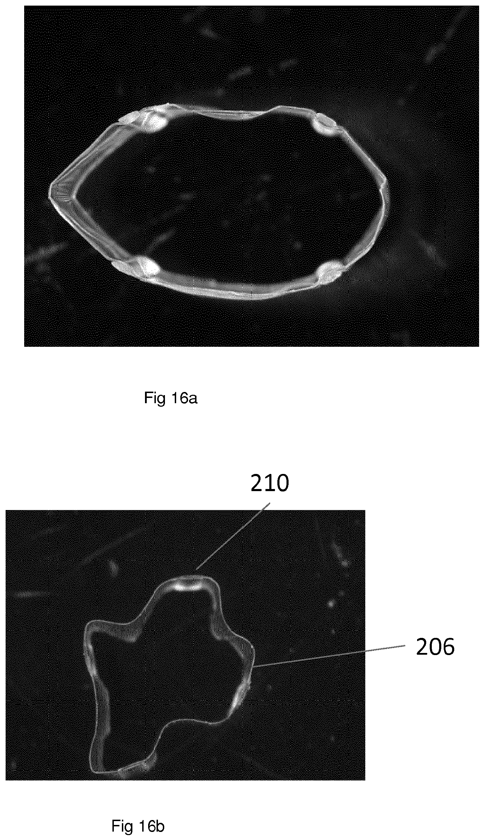

Figure 16 are photographs corresponding to the views of Figure 13.

### Description of the Preferred Embodiments

There are described below various embodiments of medical balloon which can be used in a variety of medical procedures. A number of the embodiments are to a balloon which is provided with a plurality of scoring or cutting elements extending along the outer surface of the balloon and which can be used for removing debris and in particular plaque from within a vessel, as well as for vessel dilatation. Other embodiments are directed to a balloon having strengthening elements or strips extending along the length of the balloon and which do not perform any scoring or cutting action. The strengthening elements are designed to allow an increase in the rate of inflating pressure of a balloon by reducing or eliminating the risk of circumferential tear propagation. The concepts taught herein can be used for a variety of medical balloons.

Described herein are balloons which include elongate elements including amorphous polymer material. The inventors have discovered that amorphous polymer material as described herein is particularly beneficial for making scoring or cutting or strengthening elements. In particular, the inventors have found that it provides significantly improved performance as a scoring or cutting element, which is believed to be owing to its rigidity. It can enable scoring elements to be significantly reduced in size as compared to prior art devices without any reduction in performance as compared to those prior art devices. It can also enable elements to be easily shaped, for example to flatten elements for use as strengthening elements without any scoring or cutting function. It has been found that in preferred embodiments the amorphous polymer material becomes soft and formable when heated for being formed, allowing elongate elements to be easily configured.

The person skilled in the art will appreciate that the drawings are not to scale and often depict various parts of the balloon in significantly enlarged form. Moreover, the proportions of the various elements of the balloon are not as they would be in practice. The schematic form of the drawings is intended to show clearly the different parts of the structure. A person skilled in the art will immediately appreciate, from common general knowledge, the typical dimensions and relative proportions of the various elements of the balloon, and also that these will also vary in dependence upon the specific medical application.

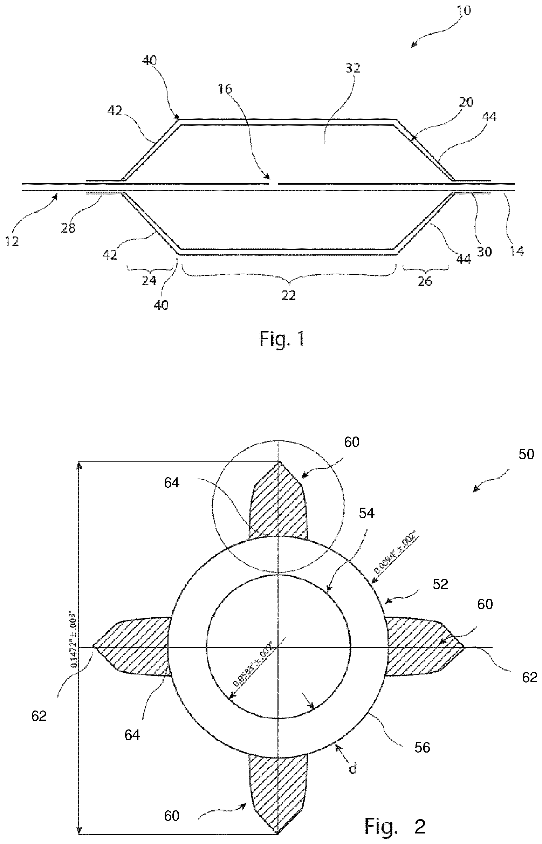

Referring now to Figure 1, this shows in schematic form and in side elevation an embodiment of medical balloon 10 shown in cross-section. The balloon 10 is carried on a balloon catheter 12 of conventional form, which terminates at a distal end 14. The balloon catheter 12 includes one or more apertures 16 in its wall, communicating with the catheter lumen, for providing inflation fluid into the balloon 20, as well as for exhausting fluid from the balloon 20 in order to deflate this for removal purposes.

In the example shown in Figure 1 the balloon 20 has a generally conventional form with, in this embodiment, a body element 22 of substantially cylindrical shape and which is of elongate form, that is has a length greater than its diameter. A person skilled in the art will appreciate that medical balloons may have a variety of different shapes and they may sometimes be wider than they are longer.

The balloon 20 also includes end portions 24, 26, which are typically conical as shown in Figure 1. Beyond the end cones 24, 26, the balloon 20 has neck portions 28, 30 which are fixed to the balloon catheter 12 in fluid tight manner, so that the balloon 20 has at least one chamber 32 which is sealed, save for the fluid connection through the aperture 16 of the balloon catheter 12.

The example of balloon 10 shown in Figure 1 is a scoring or cutting balloon and in this regard is provided with a plurality of elongated scoring or cutting elements 40, which preferably extend for the whole length of the balloon 20 and most preferably also along the length of the end cones 24, 26 and of the necks 28, 30. Having scoring elements 40 of this nature can provide longitudinal strength to the balloon 20 and, as explained below, can provide increased tear resistance, specifically by providing a mechanism for inhibiting or substantially reducing the risk of circumferential tear propagation.

The scoring elements 40 may extend longitudinally along the balloon 20, that is in a direction parallel to the longitudinal axis of the balloon 20, which could be equated with the longitudinal axis of the balloon catheter 12 as viewed in Figure 1. It is not excluded, however, that the scoring elements 40 could extend at an angle to this and may, for example, run helically along and around the balloon 20. The actual disposition of the scoring or cutting elements 40 on the balloon, as well as the number of scoring elements 40 provided on the balloon, will typically vary dependent on the medical procedure and the vessel size in which the balloon is to be used. This will be within the understanding and knowledge of the person of average skill in the art.

In Figure 1, the scoring or cutting elements 40 are shown as having a substantially uniform height along the body portion 22 of the balloon 20, although they may have a varying height in some embodiments, for example to have discontinuities or teeth along the lengths of the scoring elements 40, useful in the breaking up of plaque within a vessel. The portions 42, 44 of the scoring elements which extend along the end cones 24, 26 of the balloon 20 preferably have heights substantially less than their height along the main body portion 22 of the balloon 20. In embodiments where the scoring elements 40 extend all the way into the neck portions 28, 30 also, the scoring elements preferably have a reduced height at the necks also and in practice preferably as little as possible. It is likewise preferred that the portions of the scoring elements 40 extending along the end cones is also minimised. Given that the end cones 24, 26 and the necks 28, 30 of the scoring elements 40 will generally provide no substantial scoring or cutting function, is not necessary for them to protrude from the balloon wall at all or by any significant amount and it is also not necessary for them to have pointed ends or edges. It is preferred, in fact, that the portions of the scoring elements 40 which extend along the ends cones 24, 26 (that is the portions 42, 44) and along the necks 28, 30 be as thin as possible as this enhances the flexibility of the balloon 20, particularly when the balloon is in a deflated and folded condition, which it would be during endoluminal delivery of the balloon to the treatment site in the patient.

As will be apparent from the description and drawings which follow, in embodiments having scoring or cutting elements 40, these preferably have an outermost point or apex for use in scraping or cutting plaque from within a vessel wall. It is not excluded, though, that the scoring elements could have a rounded extremity. In all the preferred embodiments, the scoring elements will extend by an operative height beyond the outer surface of the balloon wall so as to present a discontinuity in the outer surface of the balloon. As explained below, the scoring elements 40 are also preferably made of an amorphous polymer material which is more rigid than the material of the balloon wall.

Although Figure 1 shows only two scoring elements 40, it is to be understood that there will typically be provided more than two scoring elements on the balloon 20, for example four, six or more, or any number therebetween. The actual number can be selected by the person skilled in the art, typically in dependence upon the condition and type of vessel to be treated. It is also feasible that in some embodiments, in addition to scoring elements extending longitudinally along the balloon 20, there may be provided scoring elements which extend at an angle thereto. For the reasons indicated below, though, it is preferred that the scoring elements 40 extend along the length of the balloon and substantially parallel to the longitudinal axis of the balloon 20.

It will be appreciated that the scoring elements 40 will be spaced circumferentially around the balloon 20, and in particular the body portion of the balloon 20. They are preferably equally spaced around the balloon 20.

In the embodiments described with reference to Figures 2-11 below, the balloon wall is depicted as being a single layer of material. This is, however, not essential as the balloon wall could be formed of a plurality of layers of balloon wall material. These layers may have different characteristics and have different functions.

Figure 2 shows an embodiment of raw balloon tubing. The balloon raw tubing is a precursor to the medical balloon and is typically inflated in a mold at high temperature to form the medical balloon. The mold will have internal walls with a shape equivalent to the desired final shape of the balloon. Thus, the mold may have a shape equivalent to the balloon shape shown in the example of Figure 1, or any other balloon shape desired to be formed.

Figure 2 shows an embodiment of raw balloon 50 with ribs 60 which will form the scoring elements or strengthening elements of the balloon. More specifically, referring to Figure 2, this shows in transverse cross-section an embodiment of raw balloon form 50 which has a tubing portion 52 which will form the balloon wall once the raw tubing is inflated in a suitable mold. The tubing portion 52 has an inner surface 54, which forms the inner surface of the balloon, and an outer surface 56 which will form the outer surface of the balloon. The raw tubing has a wall thickness d, which will reduce as the raw tube 50 is inflated in the mold, as the skilled person will be fully aware. The tubing portion 52 could be made of a single layer of material, as in the example shown, or could be made of a plurality of layers of the same material or materials having different constituents and characteristics.

The raw tubing 50 also includes, in this example, four ribs 60, which extend linearly along the tubing and are equally spaced circumferentially around the tubing portion 52, so as to be equidistant from one another. Each rib 60 includes an apex or pointed extremity 62, which may be uniform along the entire length of the ribs 60, but which in other embodiments may be non-uniform so as to give the ribs 60 a non-smooth, for instance a castellated or toothed, form. The ribs 60 each have a base 64 which extends to the outer surface 56 of the tubing portion but not into the tubing portion 52, as will be apparent in particular from Figure 2. In other words, the ribs are on the outer surface of the tubing portion 52, extending radially away from the tubing portion and not into the tubing portion. As a result of this disposition of the ribs 60, the outer surface 56 of the tubing portion 52 is discontinuous around the circumference of the tubing portion 52. In other words, the outer surface of the balloon 50 comprises portions of tubing 52 and the ribs 60.

The base 64 of each rib 60 is coincident with the outer surface 56 of the tubing portion. The base 64 of each rib is therefore curved in a circumferential direction with a radius of curvature which is the same or substantially the same as the radius of curvature of the outer surface 56 of the tubing portion 52.

In other words, the balloon raw tubing 50 is provided with a tubing portion 52 having an inner wall surface 54 and an outer wall surface 56, with a wall depth or thickness d. The raw tubing 50 also includes, in this example, four ribs 60 extending in the longitudinal direction of the tubing portion 52 and which are provided with apices 62 at their extremities, which are pointed, and bases 64 which extend to the position of the outer wall surface 56 of the tubing portion 52. In other words, the ribs 60 do not extend, in this example, into the wall of the tubing portion 52. This embodiment is used particularly for the production of a medical balloon having scoring or cutting elements.

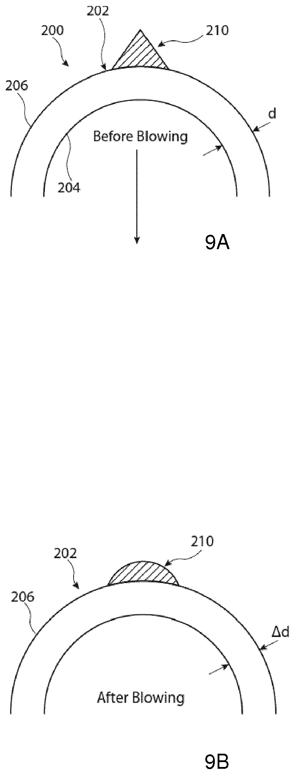

The ribs are preferably also configured so that once the balloon is fully formed and inflated, the ribs are thicker than the balloon wall. This feature and its advantages are described in more detail below, especially in connection with Figures 9 to 11.

The ribs include amorphous polymer material. Amorphous polymer material has been found to be able to provide rigidity to the ribs, enabling them to serve as effective cutting or scoring elements.

A particular advantage of using amorphous polymer material in scoring or cutting elements is that the rigidity of amorphous polymer material can improve the performance of the scoring or cutting elements by over 50%. It can therefore be possible to halve the height of the scoring or cutting elements as compared with some prior art balloons and still obtain an improved performance. Reducing the height of the scoring or cutting elements in turn reduces the profile of the balloon as a whole, making it easier to insert into small and/or tortuous vessels. Grilamid TR 55 is one example of amorphous polymer material which can obtain this improved performance.

The ribs of the raw tubing described in connection with Figure 2 and similarly in all of the embodiments described herein are preferably made of materials having different characteristics to the material or materials of which the tubing portion is made. In one embodiment, for instance, the ribs are made of an amorphous polymer material which has a greater rigidity than the rigidity of the material or materials forming the tubing portion. In yet another embodiment, the ribs may be formed of or include radiopaque and/or echogenic material, whereas the tubing portion is formed of a material which is substantially radio transparent. The ribs may have any one of or any combination of these characteristics and in the preferred embodiment are made of material which has greater rigidity.

It is preferred that the ribs and tubing portions are made from or include polymer materials which are compatible with one another. In particular, the ribs and tubing portions are preferably made of polymer materials which are able to blend and mix with one another without any noticeable interface or transition point once formed, particularly by extrusion. For this purpose, the ribs and tubing portions may be made of the same polymer type or even of the same polymer. Preferably the ribs are made of an amorphous polymer material, preferably an amorphous polyamide material, and the tubing portions are made of a polymer material of the same type or even including the same polymer but which is not amorphous.

Amorphous polymer material has been found often not to be as strong as non-amorphous polymer material, so providing the tubing portions of a non-amorphous polymer material means that the ribs can provide the rigidity desired for cutting or scoring while the tubing material can provide strength to the balloon.

In one embodiment one or the other or both of the materials of the ribs and tubing portions is provided with an additive, a structural modification and/or a blend which modifies its characteristics relative to the other material. For instance, the material forming the ribs may have an additive which increases the rigidity, rupture strength, radiopacity and/or echogenicity of the material forming the ribs in comparison with the material or materials of which the tubing portion is made. In other embodiments, the ribs may be formed of a material which is structurally modified compared to that of the tubing portions, for example by a greater degree of cross-linking or other modification which will be apparent to the person skilled in the art.

In one embodiment, the tubing portion is made of non-amorphous Nylon 12, and the ribs are made from amorphous Nylon 12. One example of amorphous Nylon 12 is Grilamid TR55.

It has been found that amorphous polymers, in particular amorphous Nylon 12, can be particularly rigid and easily shaped; they can be more rigid than their non-amorphous counterparts and can easily be shaped by a mould into the desired rib configuration.

In another embodiment, the tubing portion is made of a polyamide such as Nylon 12 and the ribs are made of an amorphous material with a different or different form of polyamide such as amorphous Nylon 66 or Nylon 6. Nylon 66 and Nylon 6 have a greater rupture strength and are more rigid than Nylon 12. Being of the same polymer type the tubing portions and ribs will co-extrude as a unitary material without any weakened transition point between two materials. In addition, as described above, the ribs can be formed of a mixture or blend with a radiopaque and/or echogenic material.

However, in some embodiments, the ribs can be non-echogenic and radiolucent and/or radio transparent.

In embodiments described and contemplated herein, the radiopaque and/or echogenic material or additive may be one or more of: tungsten, gold, platinum, palladium, barium or bismuth. Barium and bismuth are radiopaque; whereas tungsten, gold, platinum and palladium are both radiopaque and echogenic. Echogenic materials include PVC and fluoropolymers. These materials thus can provide good radiopacity, and/or echogenicity, and are biocompatible. Tungsten is the most preferred material as this has very good performance even when used in small amounts. Tungsten may also assist with enabling the material to be shaped. Materials which are solely echogenic can be seen by fluoroscopy techniques.

It is preferred that the ribs include between 50 and 90% by weight of radiopaque/echogenic material, more preferably, between 60 and 80% by weight of radiopaque and/or echogenic material. A layer with 80% of tungsten has been found to be particularly effective.

In terms of concentration by volume, the radiopaque/echogenic material may comprise substantially 11.4% to substantially 20.6%, more preferably substantially 13.7% to substantially 18.3%, most preferably 14.8% to 18.3% by volume.

Below is a reference regarding the effect of density of tungsten on % by volume. Tungsten has a density of 19.35.

| % by Weight | % by Volume |
|---|---|
| 20 | 1.4 |
| 30 | 2.4 |
| 40 | 3.6 |
| 50 | 5.4 |
| 60 | 7.9 |
| 70 | 11.7 |
| 80 | 18.5 |
| 90 | 33.9 |

It will be apparent that the ribs can be formed from a mix or blend of radiopaque/echogenic material and an amorphous polymeric material.

It is also envisaged that the tubing portion may be made of a blend of polymers, as can the ribs. It is preferred that the polymeric materials of the first and second materials are of the same type and co-extruded. This ensures a strong and unitary coupling of the elements to one another, and in some instances at least a seamless interface between the elements. In another embodiment, the polymeric materials of the tubing portion and the ribs are different types.

In embodiments, the die in the extruder can have grooves and can put the ribs on the tubing portion thereby extruding the raw tubing in a single process.

As explained above, in embodiments the ribs and the tubing portion are coextruded. Described herein are polymer materials suitable for the ribs. As described herein, in embodiments, the selected rib polymer material is different from material(s) used to form the tubing portion, and in some embodiments only the ribs include the selected rib polymer material.

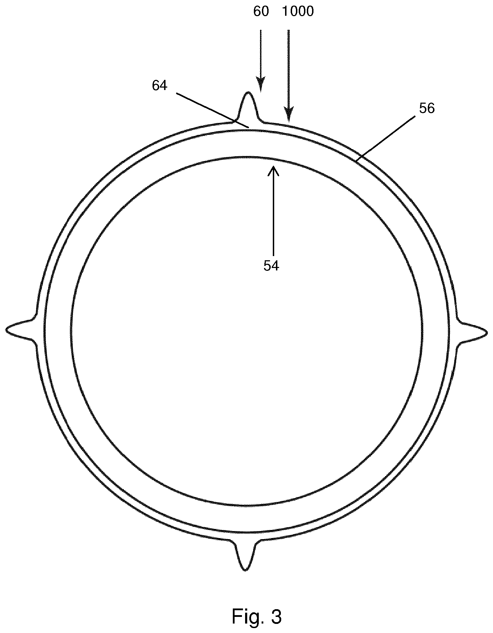

However, in other embodiments, such as shown in Figure 3, the balloon can include a layer 1000 disposed on the outer surface 56 of the tubing material. The layer 1000 is made of the selected rib polymer material. The ribs 60 are formed as part of the layer. The layer 1000 is substantially smooth circumferentially around the tubing portion with the exception of the ribs 60 which extend radially outwardly and thereby provide discontinuities in the outer surface of the layer 1000.

Providing the ribs as part of the layer 1000 has advantages in that it is easier to co-extrude the balloon in this form. However, because the layer 1000 includes amorphous polymer material, it can be weaker than the tubing portion so the sections of the layer 1000 between the ribs can adversely affect the strength of the balloon. In other words, in embodiments where only the ribs include the selected rib polymer material, the balloon can be stronger.

Although some embodiments use Nylon as the core constituent of the balloon and ribs or scoring/cutting elements, the polymeric material of the ribs and tubing portions may include one or more of polyamide, polyether block amide (Pebax), PET, polyethylene and polyurethane.

The ribs may have any of the characteristics mentioned herein.

In the embodiment of Figures 2 and 3, the ribs extend to the outer surface of the tubing portion but not into the tubing portion. This is advantageous as amorphous polymer material can be less strong than non-amorphous polymer material, and ensuring that the amorphous polymer material does not extend into the tubing portion can enable a particularly strong balloon wall to be provided.

As explained above, even though the embodiments of Figures 2 to 3 show four ribs spaced equidistantly in the circumferential direction of the tubing portion, a different number of ribs may be provided in other embodiments (whether fewer or more). The ribs need not extend precisely parallel to the longitudinal axis of the tubing portion but may, as previously explained, have different configurations, for example helical or any of the other configurations described herein.

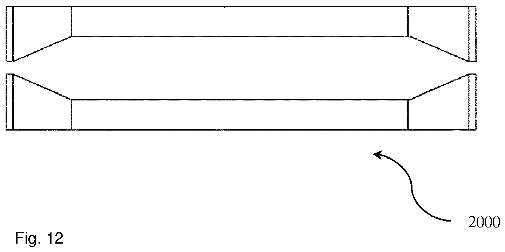

The raw tubing disclosed herein and exemplified by the embodiments of Figures 2 to 3 are used to form a medical balloon, as described, by placing the raw tubing in a suitable mold, such as mold 2000 in figure 12, and then inflating this while heating so that the tubing expands to form a balloon shape of a type similar to the medical balloon of Figure 1. In so doing, the tubing portions of the raw tubing will expand and stretch, with the thickness or depth thereof reducing and eventually being the thickness of the balloon wall. The ribs may also be stretched but typically to a far lesser extent than the tubing portion, typically as a result of the greater rigidity of the material forming the ribs. The base of the ribs will typically remain at the same relative position with respect to the balloon wall as with the raw tubing. The ribs will soften when heated despite being rigid when the balloon is used in a body vessel, because of the advantageous properties of the amorphous polymer material. The ribs can be made to retain their pointed extremities by providing suitable grooves within the mold surface to receive the ribs and ensure that they are not flattened during the blowing and heating process. As explained above, it is preferred that the ribs are flattened at the end cones of the balloon and also the necks of the balloon, the latter typically being formed of non-inflated raw tubing. Flattening can be produced within the mold, for instance by ensuring that the mold surfaces which form the conical end cones of the balloon and the ends of the mold which withhold the neck portions of the balloon, have smooth internal surfaces which will act to flatten the ribs during the balloon blowing process. As described above, reducing the height of the ribs at the conical portions of the balloon and at its necks will increase the flexibility of the balloon, particularly when the balloon is deflated and will hence increase the trackability of the balloon for endoluminal delivery purposes.

In some embodiments, as described below, the ribs may be flattened along the entirety of the length of the balloon.

Other embodiments of raw tubing and blown balloon are shown in Figure 9. These are just two embodiments from the large variety of embodiments contemplated herein, as will be apparent from the disclosure as a whole.

Figures 9A and 9B show an embodiment having similar characteristics to the example of Figure 2, in which a raw tubing 200 has a tubing portion 202 having an inner wall surface 204 and an outer wall surface 206. Attached to the outer wall surface 206 and formed preferably by co-extrusion are a plurality of ribs 210 which extend longitudinally along the raw tubing and preferably parallel to the longitudinal axis of the raw tubing. The ribs 210 (of which there may be four, fewer or more) are made of a material which is or includes an amorphous polymer material. As will be apparent from a comparison of Figures 9A and 9B, when the raw tubing 200 is blown, that is inflated while being heated in a mold, the ribs 210 will flatten (and the tubing portion 202 will stretch circumferentially and reduce in thickness to a thickness Δ *d*, in known manner). However, the ribs are configured so that after being flattened and when the balloon is inflated they have a thickness which is greater than the wall thickness, in other words greater than Δd. The thickness of a rib could also be considered to be the height of the rib. Preferably, the thickness of the ribs is at least twice, at least four times, or most preferably at least six times the thickness of the wall.

Once they have been flattened, the ribs preferably have a rounded outer surface.

An advantage of the greater thickness of the ribs is that the amorphous polymer material from which they are made can easily be shaped into the desired rounded shape so as to be smooth with the outer surface of the balloon wall. In addition, the greater thickness means that the ribs can stop circumferential tears in the balloon wall. As mentioned above, circumferential tears are potentially more problematic than longitudinal tears, and being able to stop circumferential propagation of such tears can minimise the risk of portions of a torn balloon snagging within a vessel or breaking off. The ribs 210 preferably provide a flattened outer surface which is most preferably substantially smooth with the outer wall surface 206 of the balloon 202.

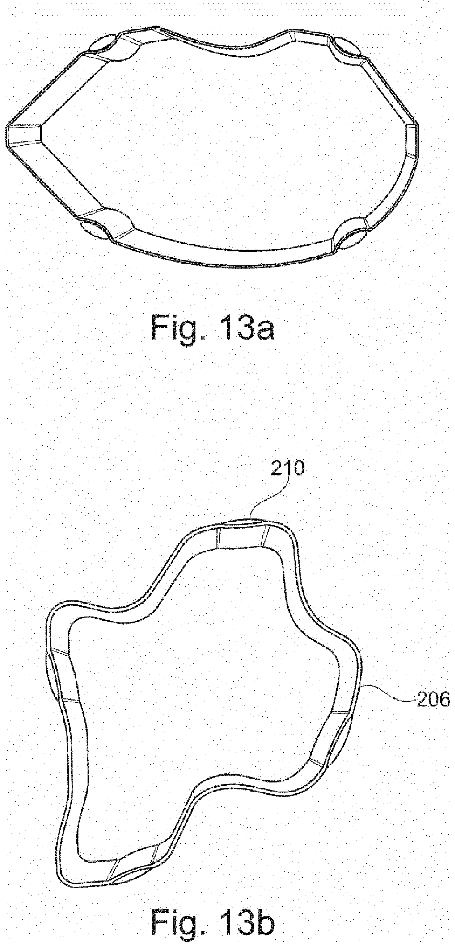

Figure 13 shows a comparison between a balloon with non-amorphous Nylon 12 ribs and a balloon with amorphous Nylon 12 ribs. Figure 13(a) shows a cross-section of a part of a balloon with non-amorphous Nylon 12 ribs, and Figure 13(b) shows a cross-section of a part of a balloon with amorphous Nylon 12 ribs 210, in particular Grilamid TR55, similar to the example of Figure 9B. As can be seen, the formability of the amorphous Nylon 12 when heated allows the ribs to be smashed into a much smoother and more continuous relationship with the balloon wall as compared to the example with non-amorphous ribs.

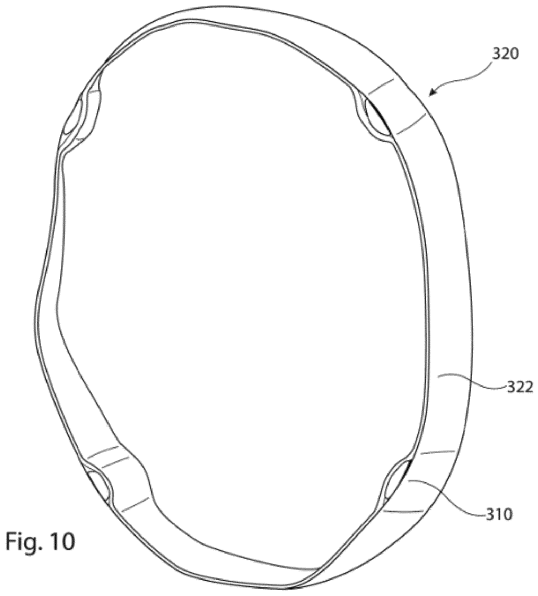

Figure 10 shows an embodiment of a balloon 320 similar to the embodiment of Figure 9B. In Figure 10, the ribs 310 of the balloon have been flattened, but the balloon is in a non-expanded state. This means that the ribs can be seen to push the balloon wall 322 radially inwards slightly. However, upon inflation, the balloon wall will expand to a substantially annular shape, pushing out the ribs 310. Figure 10 shows more clearly the increased thickness of the ribs 310 as compared to the balloon wall 322.

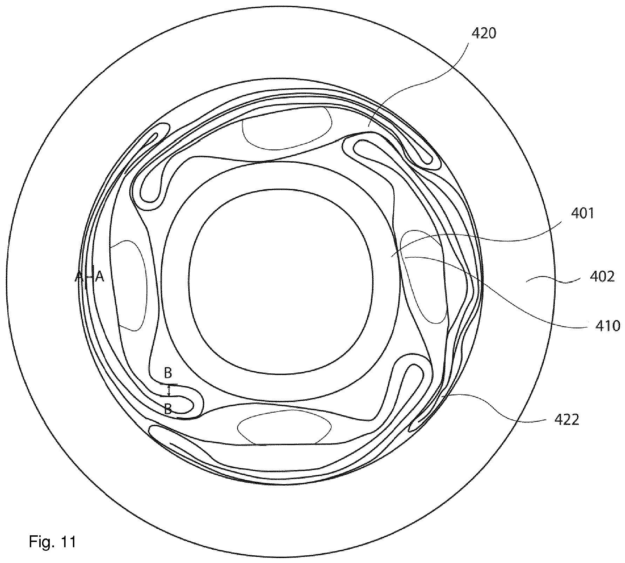

Figure 11 shows a cross section of a balloon 420 similar to the embodiment of Figure 10 folded inside a delivery device. The delivery device includes a first catheter 401 and a coaxial second catheter or sheath. The balloon is folded within an annular space between the two catheters. In this embodiment, the inner diameter of the second catheter or sheath 402 is 2.124mm (0.084in). Figure 11 also shows the greater thickness of the ribs 410 as compared to the balloon wall 422. As can be seen in Figure 11, the thickness of the balloon wall varies in this embodiment. For example, the distance A-A is 0.031mm and the distance B-B is 0.053mm.

The various embodiments of raw tubing and balloon disclosed herein provide a medical balloon with various advantages over the art. For instance, where the balloon is provided with cutting and scoring elements which are thicker than the balloon wall, these can provide resistance to or prevention of circumferential propagation of any tears to the balloon during its use. Specifically, should the balloon tear during use, for example as the result of plaque in a vessel wall or the application of too much pressure to the balloon, any such tear would tend to propagate along the balloon wall. Such a tear could propagate along the length of the balloon where there is no scoring or cutting element or other strengthening element arranged cross-wise around the balloon but this would still ensure the balloon fragments remain attached to the balloon catheter 12. On the other hand, any tears which propagate circumferentially around the balloon would eventually come up against the cutting or scoring element and be unable to propagate beyond this. The cutting or scoring element will therefore stop circumferential propagation of the tear.

Moreover, having cutting or scoring elements including amorphous polymer material and not extending into the thickness of the balloon wall means that the balloon wall can provide a strong support, especially if made from non-amorphous polymer material, while the scoring or cutting element provides the rigidity needed for providing an effective scoring or cutting action which in particular enables these to apply more cutting or scoring pressure to plaque within the vessel wall.

Increased resistance to circumferential tear propagation can also be achieved with the provision of thick flattened strengthening elements extending along the length of the balloon, whether or not such elements act as cutting or scoring elements for an angioplasty procedure. In such embodiments, as described herein, the flattening strengthening elements are disposed on the outside of the balloon wall.

The provision of radiopaque material in the cutting/scoring or strengthening elements enables the balloon to be seen during the medical procedure by standard imaging techniques although this is not provided in all embodiments. It is possible to have the radiopaque elements formed within a polymer material which is the same as or the same type, or compatible in terms of blending, as the material from which the balloon wall is made, which enables the raw tubing to be produced in a single coextrusion. This is a simpler process and can provide a balloon having better structural integrity than a balloon made in separate stages with components which are separately attached to one another.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

## Claims

1. A medical balloon (10) including:
a balloon body member (20) made of a first polymer material;
the body member having a balloon wall with a wall thickness and inner and outer wall surfaces, the body member being disposed between first and second balloon ends, the balloon wall also extending along end cones; anc **characterized in that** at least one elongate element (40) on the balloon wall extending along the body member between the first and second balloon ends and along the end cones, the at least one elongate element being made of a second polymer material, the second polymer material including an amorphous polymer material; at least a part of the or each elongate element having a flattened outer surface which is substantially smooth with the outer wall surface or which is substantially non-protruding from the balloon wall.

2. A medical balloon according to claim 1, wherein the outer surface of the or each elongate element is flattened and substantially smooth with the outer wall surface of the body member.

3. A medical balloon according to claim 1or 2, wherein the outer surface of the or each elongate element is flattened and substantially smooth with the outer wall surface, or non-protruding from the balloon wall, at the end cones.

4. A medical balloon according to any preceding claim, wherein each of the at least one elongate element is a scoring or cutting element and/or has a rounded outer surface.

5. A medical balloon according to any preceding claim, wherein each of the at least one elongate element has an inflated elongate element thickness in an inflated condition of the balloon and the balloon wall has an inflated wall thickness in an inflated condition of the balloon, and wherein the inflated elongate element thickness of each of the at least one elongate element is greater than the inflated wall thickness.

6. A medical balloon according to claim 5, wherein the inflated elongate element thickness of each of the at least one elongate element is at least twice the inflated wall thickness, at least four times the inflated wall thickness, or at least six times the inflated wall thickness.

7. A medical balloon according to any preceding claim, wherein the first polymer material includes a non-amorphous polymer material.

8. A medical balloon according to any preceding claim, wherein the second polymer material is substantially radiolucent and/or non-echogenic.

9. A medical balloon according to any preceding claim, wherein the second polymer material has at least one second physical characteristic different from at least one first physical characteristic of the first polymer material.

10. A medical balloon according to any preceding claim, wherein the first and second polymer materials are of the same polymer type.

11. A medical balloon according to any preceding claim wherein the balloon body member and the at least one elongate element are coextruded.

12. A medical balloon according to any preceding claim, wherein the first and second materials include Nylon, preferably Nylon 12.

13. A medical balloon according to any preceding claim, including a plurality of said elongate elements, optionally spaced from one another circumferentially around the balloon body member.

14. A medical balloon according to any preceding claim, wherein the at least one elongate element extends generally linearly between the first and second ends; and/or
wherein the at least one elongate element extends along the first and second ends of the balloon and is flattened at the first and second ends.

15. A method of forming a medical balloon including the steps of:
forming a raw balloon tubing by:
a) forming a tubing portion made of a first polymer material;
b) applying on the tubing portion at least one elongate element made of a second polymer material, the second polymer material including an amorphous polymer material;
placing the raw balloon tubing in a mold;
heating and inflating the raw balloon tubing in the mold to flatten at least a part of the or each elongate element and to provide the at least a part of the or each elongate element with an outer surface which is substantially smooth with an outer wall surface of the balloon or substantially non-protruding from a balloon wall.

## Patentansprüche

1. Medizinischer Ballon (10), welcher einschließt:
ein Ballonkorpuselement (20), das aus einem ersten Polymermaterial gefertigt ist;
wobei das Korpuselement eine Ballonwand mit einer Wanddicke und Innen- und Außenwandflächen aufweist, wobei das Korpuselement zwischen dem ersten und dem zweiten Ballonende angeordnet ist, wobei sich die Ballonwand auch entlang der Endkegel erstreckt; und **dadurch gekennzeichnet, dass** mindestens ein längliches Element (40) auf der Ballonwand sich entlang des Korpuselements zwischen dem ersten und dem zweiten Ballonende und entlang der Endkegel erstreckt, wobei das mindestens eine längliche Element aus einem zweiten Polymermaterial gefertigt ist, wobei das zweite Polymermaterial ein amorphes Polymermaterial einschließt; und mindestens ein Teil von dem oder jedem länglichen Element eine abgeflachte Außenfläche aufweist, die im Wesentlichen nahtlos mit der Außenwandfläche ist, oder die im Wesentlichen nicht aus der Ballonwand hervorragt.

2. Medizinischer Ballon nach Anspruch 1, wobei die Außenfläche von dem oder jedem länglichen Element abgeflacht und im Wesentlichen nahtlos mit der Außenwandfläche des Korpuselements ist.

3. Medizinischer Ballon nach Anspruch 1 oder 2, wobei an den Endkegeln die Außenfläche von dem oder jedem länglichen Element abgeflacht und im Wesentlichen nahtlos mit der Außenwandfläche des Korpuselements ist oder nicht aus der Ballonwand hervorragt.

4. Medizinischer Ballon nach einem der vorhergehenden Ansprüche, wobei jedes von dem mindestens einen länglichen Element ein Ritz- oder Schneidelement (Scoring- oder Cutting-Element) ist und/oder eine abgerundete Außenfläche aufweist.

5. Medizinischer Ballon nach einem der vorhergehenden Ansprüche, wobei jedes von dem mindestens einen länglichen Element eine Dicke des aufgeblasenen länglichen Elements in einem aufgeblasenen Zustand des Ballons aufweist und die Ballonwand eine aufgeblasene Wanddicke in einem aufgeblasenen Zustand des Ballons aufweist, und wobei die Dicke des aufgeblasenen länglichen Elements von jedem des mindestens einen länglichen Elements größer als die aufgeblasene Wanddicke ist.

6. Medizinischer Ballon nach Anspruch 5, wobei die Dicke des aufgeblasenen länglichen Elements von jedem von dem mindestens einen länglichen Element mindestens das Doppelte der aufgeblasenen Wanddicke, mindestens das Vierfache der aufgeblasenen Wanddicke oder mindestens das Sechsfache der aufgeblasenen Wanddicke beträgt.

7. Medizinischer Ballon nach einem der vorhergehenden Ansprüche, wobei das erste Polymermaterial ein nicht-amorphes Polymermaterial einschließt.

8. Medizinischer Ballon nach einem der vorhergehenden Ansprüche, wobei das zweite Polymermaterial im Wesentlichen strahlendurchlässig und/oder anechogen ist.

9. Medizinischer Ballon nach einem der vorhergehenden Ansprüche, wobei das zweite Polymermaterial mindestens ein zweites physikalisches Charakteristikum aufweist, das sich von mindestens einem ersten physikalischen Charakteristikum des ersten Polymermaterials unterscheidet.

10. Medizinischer Ballon nach einem der vorhergehenden Ansprüche, wobei das erste und zweite Polymermaterial vom gleichen Polymertyp sind.

11. Medizinischer Ballon nach einem der vorhergehenden Ansprüche, wobei das Ballonkorpuselement und das mindestens eine längliche Element coextrudiert sind.

12. Medizinischer Ballon nach einem der vorhergehenden Ansprüche, wobei das erste und zweite Material Nylon einschließen, vorzugsweise Nylon 12.

13. Medizinischer Ballon nach einem der vorhergehenden Ansprüche, der eine Vielzahl der länglichen Elemente einschließt, die gegebenenfalls im Umfang um das Ballonkorpuselement herum beabstandet sind.

14. Medizinischer Ballon nach einem der vorhergehenden Ansprüche, wobei das mindestens eine längliche Element sich allgemein linear zwischen dem ersten und dem zweiten Ende erstreckt; und/oder wobei das mindestens eine längliche Element sich entlang des ersten und zweiten Endes des Ballons erstreckt und an dem ersten und dem zweiten Ende abgeflacht ist.

15. Verfahren zum Bilden eines medizinischen Ballons, das die Schritte einschließt:
Bilden eines Rohballonschlauchs durch:
a) Bilden eines Schlauchanteils, der aus einem ersten Polymermaterial gebildet ist;
b) Aufbringen von mindestens einem länglichen Element, das aus einem zweiten Polymermaterial gefertigt ist, auf den Schlauchanteil, wobei das zweite Polymermaterial ein amorphes Polymermaterial einschließt;
Platzieren des Rohballonschlauchs in einer Form;
Erwärmen und Aufblasen des Rohballonschlauchs in der Form, um mindestens einen Teil von dem oder jedem länglichen Element abzuflachen und den mindestens einen Teil von dem oder jedem länglichen Element mit einer Außenfläche auszustatten, die im Wesentlichen nahtlos mit einer Außenwandfläche des Ballons ist oder im Wesentlichen aus einer Ballonwand nicht hervorragt.

## Revendications

1. Ballonnet médical (10) comprenant :
un élément de corps (20) de ballonnet constitué d'un premier matériau polymère ;
l'élément de corps comportant une paroi de ballonnet ayant une épaisseur de paroi et des surfaces interne et externe de paroi, l'élément de corps étant disposé entre des première et seconde extrémités de ballonnet, la paroi de ballonnet s'étendant également le long de cônes terminaux ; et **caractérisé en ce que**
au moins un élément allongé (40) sur la paroi de ballonnet s'étend le long de l'élément de corps entre les première et seconde extrémités de ballonnet et le long des cônes d'extrémité, l'au moins un élément allongé étant constitué d'un second matériau polymère, le second matériau polymère comprenant un matériau polymère amorphe ; au moins une partie du ou de chaque élément allongé ayant une surface externe aplatie qui est sensiblement uniforme avec la surface de paroi externe ou qui est sensiblement non saillante depuis la paroi de ballonnet.

2. Ballonnet médical selon la revendication 1, dans lequel la surface externe du ou chaque élément allongé est aplatie et sensiblement uniforme avec la surface de paroi externe de l'élément de corps.

3. Ballonnet médical selon la revendication 1 ou 2, dans lequel la surface externe du ou de chaque élément allongé est aplatie et sensiblement uniforme avec la surface de paroi externe, ou non saillante depuis la paroi de ballonnet, au niveau des cônes terminaux.

4. Ballonnet médical selon l'une quelconque des revendications précédentes, dans lequel chacun parmi l'au moins un élément allongé est un élément de rainurage ou de coupe et/ou a une surface externe arrondie.

5. Ballonnet médical selon l'une quelconque des revendications précédentes, dans lequel chacun parmi l'au moins un élément allongé a une épaisseur d'élément allongé gonflé dans un état gonflé du ballonnet et la paroi de ballonnet a une épaisseur de paroi gonflée dans un état gonflé du ballonnet, et dans lequel l'épaisseur d'élément allongé gonflé de chacun parmi l'au moins un élément allongé est supérieure à l'épaisseur de paroi gonflée.

6. Ballonnet médical selon la revendication 5, dans lequel l'épaisseur d'élément allongé gonflé de chacun parmi l'au moins un élément allongé est au moins deux fois l'épaisseur de paroi gonflée, au moins quatre fois l'épaisseur de paroi gonflée, ou au moins six fois l'épaisseur de paroi gonflée.

7. Ballonnet médical selon l'une quelconque des revendications précédentes, dans lequel le premier matériau polymère comprend un matériau polymère non amorphe.

8. Ballonnet médical selon l'une quelconque des revendications précédentes, dans lequel le second matériau polymère est sensiblement radiotransparent et/ou non échogène.

9. Ballonnet médical selon l'une quelconque des revendications précédentes, dans lequel le second matériau polymère a au moins une seconde caractéristique physique différente d'au moins une première caractéristique physique du premier matériau polymère.

10. Ballonnet médical selon l'une quelconque des revendications précédentes, dans lequel les premier et second matériaux polymères sont du même type de polymère.

11. Ballonnet médical selon l'une quelconque des revendications précédentes dans lequel l'élément de corps de ballonnet et l'au moins un élément allongé sont coextrudés.

12. Ballonnet médical selon l'une quelconque des revendications précédentes, dans lequel les premier et second matériaux comprennent du nylon, de préférence du nylon 12.

13. Ballonnet médical selon l'une quelconque des revendications précédentes, comprenant une pluralité desdits éléments allongés, éventuellement espacés les uns des autres circonférentiellement autour de l'élément de corps de ballonnet.

14. Ballonnet médical selon l'une quelconque des revendications précédentes, dans lequel l'au moins un élément allongé s'étend généralement linéairement entre les première et seconde extrémités ; et/ou
dans lequel l'au moins un élément allongé s'étend le long des première et seconde extrémités du ballonnet et est aplati au niveau des première et seconde extrémités.

15. Procédé de formation d'un ballonnet médical comprenant les étapes de :
formation d'un tube de ballonnet brut en :
a) formant une partie tube constituée d'un premier matériau polymère ;
b) appliquant sur la partie tube au moins un élément allongé constitué d'un second matériau polymère, le second matériau polymère comprenant un matériau polymère amorphe ;
placement du tube de ballonnet brut dans un moule ;
chauffage et gonflage du tube de ballonnet brut dans le moule pour aplatir au moins une partie du ou de chaque élément allongé et pour fournir à l'au moins une partie du ou de chaque élément allongé une surface externe qui est sensiblement uniforme avec une surface de paroi externe du ballonnet ou sensiblement non saillante depuis une paroi de ballonnet.
